# EUROPÄISCHE PATENTSCHRIFT

(11) **EP 3 240 520 B1**
(45) Veröffentlichungstag und Bekanntmachung des Hinweises auf die Patenterteilung: **19.12.2018**
(21) Anmeldenummer: 15825797.2
(22) Anmeldetag: 23.12.2015
(51) Int. Cl.: A61J 1/20

(54) **MISCH- UND/ODER TRANSFERVORRICHTUNG**
MIXING AND/OR TRANSFERRING DEVICE
DISPOSITIF DE MÉLANGE ET/OU DE TRANSFERT

(30) Priorität: 30.12.2014 DE 102014119712; 11.05.2015 DE 102015107312
(43) Veröffentlichungstag der Anmeldung: 08.11.2017
(73) Patentinhaber: SFM Medical Devices GmbH, 63607 Wächtersbach (DE)
(72) Erfinder: HENNINGER, Peter, 63636 Brachttal (DE); KEHR, Markus, 63599 Biebergemünd (DE)
(74) Vertreter: Stoffregen, Hans-Herbert
(86) Internationale Anmeldenummer: PCT/EP2015/081196
(87) Internationale Veröffentlichungsnummer: WO 2016/107811

(56) Entgegenhaltungen:
- DE-A1-102008 002 800
- DE-T2- 69 320 400

## Beschreibung

Die Erfindung bezieht sich auf eine Misch- und/oder Transfervorrichtung zum Mischen einer ersten Substanz und einer zweiten Substanz bzw. Überleiten zumindest einer der Substanzen, die in einem ersten bzw. zweiten Behältnis vorhanden sind, umfassend
- einen ersten Adapter mit einer zur Aufnahme des ersten Behältnisses geeigneten Umfangswandung, entlang der und von dieser zumindest abschnittsweise umgeben ein Kanülenkörper verläuft, sowie einem mit dem Kanülenkörper in Verbindung stehenden hohlzylindrischen Abschnitt,
- einen zweiten Adapter mit einer zur Aufnahme des zweiten Behältnisses geeigneten Umfangswandung, entlang der und koaxial von dieser zumindest abschnittsweise umgeben ein Kanülenkörper verläuft, sowie einem mit dem Kanülenkörper in Verbindung stehenden hohlzylindrischen Abschnitt,
wobei bei zusammengesetztem ersten und zweiten Adapter die hohlzylindrischen Abschnitte vorzugsweise flüssigkeitsabdichtend ineinander greifen und der erste und der zweite Adapter miteinander lösbar schraubbar verbunden sind.

Bei einer Mischvorrichtung nach der US-B-6,558,365 werden zum Mischen der in Behältnissen vorhandenen Fluide, von denen es sich bei einem insbesondere um eine medizinische Substanz in pulvriger Form und bei dem anderen um eine Flüssigkeit handeln kann, die Adapter zunächst in Art einer Schraubverbindung über Luer-Lock-Kegel miteinander verbunden. Sodann werden in die von Umfangswandungen gebildeten Aufnahmen die Behältnisse eingebracht, die mit durchstechbaren Kappen verschlossen sind. Da das mit Pulver teilweise gefüllte Behältnis unter Unterdruck steht, wird die in dem anderen Behältnis vorhandene Flüssigkeit angesaugt, so dass anschließend die Vermischung der Flüssigkeit mit dem Medikament erfolgen kann. Die Adapter werden sodann voneinander getrennt, um den das Behältnis mit dem Medikament aufweisenden Adapter z. B. mit einer Spritze zu verbinden. Zum einfachen Verbinden mit einer Spritze wird der Luer-Lock-Kegel benutzt.

Die diesbezügliche Konstruktion zeigt den Nachteil, dass die Verbindung zwischen den Adaptern über die mittig vorhandenen Luer-Lock-Kegel erfolgt, so dass bei Einwirken auf die einen erheblich größeren Durchmesser aufweisende Umfangswandungen der Adapter Kräfte einwirken können, die zu einem ungewollten Lösen führen. Bei zusammengesetzten Adaptern sind die Bodenwandungen zueinander beabstandet, so dass die Adapter gegeneinander bewegt und somit die Verbindung über die hohlzylindrischen Abschnitte verbogen werden kann. Auch kann es beim Zusammenschrauben der Adapter zu Verkantungen oder zu einem Bruch im Luer-Bereich kommen.

Der DE-B-10 2006 031 712 ist eine medizinische Transfervorrichtung zu entnehmen, die aus einem eine erste Flasche aufnehmenden rohrförmigen Teil und einem zu diesem axial verschiebbaren eine zweite Flasche aufnehmenden Halteteil besteht. In dem Halteteil ist ein Flansch axial verschiebbar angeordnet. Zum Verbinden der Flaschen ist es erforderlich, dass die von dem rohrförmigen Teil aufgenommene Flasche axial in diesem verschoben wird, um hierdurch von dem Teil ausgehende Laschen derart zu spreizen, dass das Halteteil zusammen mit dem Flansch innerhalb des rohrförmigen Teils verschoben werden kann.

Ein medizinisches Transfergerät nach der DE-B-10 2004 005 435 ist dreiteilig ausgebildet, wobei gleichfalls ein axiales Verschieben der Teile zueinander erforderlich ist, um die von äußeren Teilen aufgenommenen Flaschen miteinander zu verbinden.

Ein Konnektor für medizinische Flüssigkeiten nach der DE-C-100 30 474 weist ein Anschlussstück auf, das aus mittels Ultraschall verschweißten Teilstücken besteht.

Aus der EP-A-1 498 097 ist eine zwei Adapter umfassende Transfereinrichtung bekannt. Um die Adapter zu verbinden, geht von einer Umfangswandung eines Adapters eine Nut aus, in die ein von der Umfangswandung des anderen Adapters ausgehender Vorsprung eingreift.

Bei einem Transfersystem nach der WO-A-92/11897 werden Adapter eines Transfersystems durch Verschrauben miteinander verbunden.

Gegenstand der WO-A-2005/041846 ist ein Transfersystem, bei dem ein Adapter mit einem Vial und ein anderer Adapter mit einem flexiblen Behältnis verbunden sind. Die Adapter sind über ein Verbindungsstück miteinander verbunden.

Bei einer Mischvorrichtung nach der DE-B-10 2008 002 800 A1 können zum Verbinden von Adapterteilen von diesen formschlüssig ineinander greifende Elemente ausgehen.

Aufgabe der vorliegenden Erfindung ist es, eine Transfer- bzw. Mischvorrichtung der eingangs genannten Art so weiterzubilden, dass diese einfach zu handhaben ist, jedoch eine hinreichende Stabilität bietet, insbesondere ausgeschlossen ist, dass durch äußeres Erfassen der Adapterteile die von diesen aufzunehmenden Behältnisse - auch Vials genannt - ungewollt positionsverändert werden. Auch soll ggf. die Möglichkeit gegeben sein, mit einfachen Maßnahmen eine Anpassung an Behältnisse unterschiedlicher Größen vornehmen zu können.

Ein weiterer Aspekt der Erfindung soll die Möglichkeit schaffen, dass nach erfolgter Medikamentenaufbereitung eine Spritzenentnahme aus dem Vial ohne zusätzliche Kanüle ermöglicht wird.

Auch soll die Möglichkeit gegeben sein, aufbereitetes Medikament wie Lyophilisat zu filtern. Gegebenenfalls soll dies auch bezüglich der Flüssigkeit wie Wasser der Fall sein.

Die Möglichkeit, dass eine Needle-Free-Benutzung erfolgt, soll gegeben sein.

Ferner soll die Vorrichtung bis zur Medikamentenentnahme geschlossen bleiben.

Eine einfache Bedienbarkeit soll gegeben sein, so dass eine Nutzung auch von Personen mit eingeschränkter Motorik ermöglicht wird.

Zur Lösung der Aufgabe bzw. von Aspekten ist im Wesentlichen vorgesehen, dass der erste und der zweite Adapter jeweils einen hohlzylinderförmigen Außenkörper mit Außenumfangswandung und eine senkrecht zur Längsachse des Außenkörpers verlaufende Zwischenwandung aufweist, dass bei zusammengesetzten Adaptern ein ein Innengewinde aufweisender Abschnitt der Außenumfangswandung des einen Adapters einen ein Außengewinde aufweisenden Abschnitt der Umfangswandung des anderen Adapters bei ineinander greifenden Gewindeabschnitten umgibt und dass der hohlzylinderförmige Außenkörper des ersten und/oder des zweiten Adapters einen Einsatz mit der das erste oder zweite Behältnis aufnehmenden Umfangswandung aufweist.

Hohlzylinderförmig bedeutet, dass grundsätzlich eine Hohlzylindergeometrie gegeben sein soll, gleichwenn der Außenkörper außenseitig auch eine hiervon abweichende Geometrie aufweisen kann. Insbesondere schließt der Begriff hohlzylinderförmiger Außenkörper auch einen solchen ein, dessen Außengeometrie z. B. die einer Mehrkantsäule wie Quaderform aufweist. Insoweit ist hohlzylinderförmig als Synonym zu verstehen. Ein hohlzylinderförmiger Außenkörper soll jedoch insbesondere vermitteln, dass der Außenkörper innenseitig eine Zylindergeometrie aufweist.

Insbesondere ist vorgesehen, dass jeder Adapter einen Einsatz mit der das erste bzw. zweite Behältnis aufnehmenden Umfangswandung aufweist.

Abweichend von vorbekannten und gattungsbildenden Konstruktionen, bei denen die Adapter zum Verbinden miteinander verschraubt werden, ist für das Behältnis eine gesonderte Aufnahme vorgesehen, die sich innerhalb des quasi als Gehäuse zu bezeichnenden hohlzylinder- bzw. hohlkörperförmigen Außenkörpers befindet, so dass dieser aus einem relativ steifen Material, insbesondere Kunststoffmaterial bestehen kann, der seine Geometrie beim Erfassen des Außenkörpers beibehält. Nach dem Stand der Technik ist demgegenüber vorgesehen, dass der Adapter umfangsseitig durch Schlitze getrennte Abschnitte aufweist, um ein Vial aufzunehmen, so dass die Adapterteile umfangsseitig flexibel sind.

Dadurch, dass ein Einsatz zur Aufnahme des Vials vorgesehen ist, ergibt sich ein modularer Aufbau mit der Folge, dass ohne Veränderung der Geometrie des äußeren Teils, also des hohlzylinderförmigen Außenkörpers, Einsätze unterschiedlicher Querschnitte eingebracht werden können, um an Vials unterschiedlicher Abmessungen angepasst zu sein, um diese also im erforderlichen Umfang fixieren zu können.

Die hohlzylinder- bzw. hohlkörperförmigen Außenkörper der Adapter bilden ein Gehäuse, das zumindest abschnittsweise außenseitig eine Zylinder- und/oder Quadergeometrie aufweisen kann. Eine Zylinderaußengeometrie weist insbesondere der Adapter auf, der ein Vial mit einer medizinischen Substanz aufnimmt. Der andere Adapter kann eine quaderförmige Außengeometrie aufweisen. Unabhängig hiervon ist es nicht zwingend erforderlich, dass die Außenflächen bündig ineinander übergehen, wenngleich dies zumindest abschnittsweise möglich ist.

Gegebenenfalls kann zwischen den Adapterteilen ein Spalt oder Schlitz oder eine umlaufende Einbuchtung vorhanden sein, wenn diese zusammengesetzt, also miteinander verschraubt sind. Ungeachtet dessen ist eine erforderliche Stabilität der Adapterteile und ein Verkanten der Adapterteile zueinander ausgeschlossen, da diese abschnittsweise ineinander greifen. Zusätzlich kann eine Stirnfläche eines Adapters sich entlang eines Absatzes des anderen Adapters erstrecken bzw. zu diesem in einem Abstand ausgerichtet sein, dass dem Grunde nach ein Verkanten ausgeschlossen ist.

Durch den modularen Aufbau, also des in den äußeren hohlzylinder- bzw. hohlkörperförmigen Körper einsetzbaren mit diesem insbesondere stoffschlüssig zu verbindenden Einsatz ergibt sich des Weiteren der Vorteil, dass zwischen Bodenwandung des Einsatzes und Zwischenwandung des hohlzylinder- bzw. hohlkörperförmigen Außenkörpers problemlos ein Filterelement positioniert und fixiert werden kann. Dieses kann zuvor mit der Außenfläche der Bodenwandung des Einsatzes verbunden wie verschweißt sein, um sodann den Einsatz mit der Zwischenwandung des hohlzylinder bzw. hohlkörperförmigen Außenkörpers insbesondere mittels Ultraschallschweißens stoffschlüssig derart zu verbinden, dass die Bodenwandung mit der Zwischenwandung flüssigkeitsdicht verbunden ist. Somit ist sichergestellt, dass bei verbundenen Adaptern die durch den insbesondere als Luer-Kegel bzw. Luer-Lock-Kegel ausgebildeten weiblichen und als Luer ausgebildeten männlichen hohlzylindrischen Abschnitt strömende Flüssigkeit ausschließlich über das Filter von einem Vial in das andere Vial strömen kann. Dies erfolgt insbesondere entsprechend dem Stand der Technik dadurch, dass das die medizinische Substanz wie Pulver enthaltende Vial unter Unterdruck steht, so dass nach Herstellung der Verbindung mit dem anderen Vial die in diesem vorhandene Flüssigkeit angesaugt werden kann.

Sind Filter vorhanden, sollten diese als Flächenfilter ausgebildet sein. Dabei wird Filtergewebe zwischen zwei Flächen platziert. Zur gleichmäßigen Verteilung der Flüssigkeit kann die komplette Oberfläche, entlang der sich das Filtergewebe erstreckt, mit regelmäßigen zentrisch verlaufenden Vertiefungen versehen sein. Die Zu- wie auch Ableitung der Flüssigkeit erfolgt vorzugsweise über vier symmetrisch angeordnete zentral verlaufende Kanäle, die die Flüssigkeit zu zentrischen Vertiefungen führt. Die Vorsprünge in der Oberfläche dienen als Abstützung des Gewebes und somit als Auflagefläche.

Durch den weiblichen Luer-Kegel wie Luer-Lock-Kegel ist eine problemlose Entnahme des zubereiteten Medikamentes möglich, ohne dass eine Metallkanüle erforderlich ist. Somit ist eine Needle-Free-Entnahme gegeben.

Durch das außenliegende Gewinde, über das die beiden Adapter verbunden werden, ist eine extreme Steifigkeit gegeben. Das Handling wird hierdurch erleichtert, insbesondere beim Aufsetzen des Vials. Gleichzeitig wird die Luer-Verbindung vor Beschädigungen und Leckage geschützt.

Der modulare Aufbau ermöglicht die Kombination unterschiedlicher Vialgrößen.

Ferner besteht die Möglichkeit, dass von dem Adapter, in dem das Vial mit der medizinischen Substanz wie Lyophilisat aufgenommen wird, ein oder mehrere Vorsprünge ausgehen, der bzw. die ein Wegrollen bei einer unachtsamen Bedienung verhindert. Anstelle eines Vorsprungs kann zumindest einer der Adapter z. B. eine von einer Zylindergeometrie abweichende Außengeometrie wie quaderförmige Geometrie aufweisen.

Zum Fixieren des Behältnisses ist insbesondere vorgesehen, dass die insbesondere konzentrisch von der Außenumfangswandung des Außenkörpers wie Gehäuse umgebene Umfangswandung des Einsatzes radial sich ins Innere des Einsatzes erstreckenden Vorsprünge zum Halten des Behältnisses aufweist, wobei die Umfangswandung zumindest im Bereich der Vorsprünge beabstandet zur Innenseite der Außenumfangswandung verläuft.

Dabei dienen die Vorsprünge insbesondere dazu, einen wulstartigen Rand des Vials zu hinterfassen. Damit die elastisch verbiegbaren Vorsprünge, die von Wandabschnitten der Umfangswandung ausgehen, im erforderlichen Umfang verstellt werden können, ist die Umfangswandung zumindest im Bereich der Vorsprünge zur Außenumfangswandung beabstandet. Insbesondere ist vorgesehen, dass die Umfangswandung insgesamt beabstandet zur Außenumfangswandung verläuft.

Um ein einfaches Ausrichten des Einsatzes zu dem Adapter zu ermöglichen, ist in Weiterbildung der Erfindung vorgesehen, dass die z. B. als Luer-Kegel ausgebildeten hohlzylindrischen Abschnitte von den Zwischenwandungen der Adapter ausgehen. Demgegenüber erstreckt sich der vorzugsweise als Kunststoffspike ausgebildete auch als Kanülenkörper zu bezeichnende Hohlnadelkörper entgegengerichtet verlaufend zu dem hohlzylindrischen Abschnitt von der Bodenwandung des zugeordneten Einsatzes.

Hierdurch ist jedoch nicht die Erfindung eingeschränkt. Selbstverständlich bestünde auch die Möglichkeit, dass z. B. der hohlzylindrische Abschnitt und der Hohlnadelkörper von der Bodenwandung oder der Zwischenwandung ausgehen.

Um eine Drosselung für die durchströmende Flüssigkeit zu bilden, kann die erfindungsgemäße Mischvorrichtung derart weitergebildet sein, dass zumindest in einem der Adapter der bodenseitig bzw. zwischenwandseitig verlaufende Querschnitt des Kanülenkörpers kleiner ist als der Querschnitt der von der bodenseitig- bzw. zwischenwandseitig verlaufenden Verbindungsöffnung, die die Verbindung zu dem hohlzylindrischen Abschnitt wie Luer-Kegel bildet.

Die Drosselung sollte in dem Adapter vorgesehen sein, der das die Flüssigkeit enthaltende Vial aufnimmt.

Das insbesondere als flächiges Filterelement ausgebildete Filter erstreckt sich zwischen der Bodenwandung und der Zwischenwandung des Adapters, wobei insbesondere jeder Adapter ein entsprechendes Filterelement aufweist.

Die außenseitige Bodenfläche des Einsatzes, entlang der sich das Filter erstreckt, sollte strukturiert sein, so dass ein flächiges Anliegen des Filterelementes vermieden wird.

Der eine Drosselung bewirkende querschnittsmäßige Sprung zwischen hohlzylindrischem Abschnitt wie Luer-Kegel und Kanülenkörper wie Kunststoffspike ist dabei nur in dem Adapter erforderlich, der mit dem die Flüssigkeit enthaltenden Vial verbunden ist.

In Weiterbildung sieht die Erfindung vor, dass der Einsatz einen bodenseitig verlaufenden zylindrischen ersten Abschnitt und einen über eine Stufe übergehenden und die Umfangswandung bildenden zweiten Abschnitt größeren Außendurchmessers umfasst, und dass die Stufe außenseitig auf einem von der Zwischenwandung des hohlzylindrischen Außenkörpers axial abragenden Vorsprung abgestützt ist.

Unabhängig hiervon ist insbesondere vorgesehen, dass der Einsatz mit dem hohlkörperförmigen Außenkörper umlaufend flüssigkeitsdicht verbunden ist, wobei insbesondere eine stoffschlüssige Verbindung vorliegt, die mittels Ultraschallschweißens hergestellt werden kann.

Bezüglich der hohlzylindrischen die Flüssigkeitsverbindung herstellenden Abschnitte ist vorgesehen, dass von der Zwischenwandung des das Innengewinde aufweisenden hohlkörperförmigen Außenkörpers ein männlicher hohlzylindrischer Abschnitt, wie männlicher Luer-Kegel, abragt, der in einen von der Zwischenwandung des das Außengewinde aufweisenden hohlzylinderförmigen Außenkörpers abragenden weiblichen hohlzylindrischen Abschnitt, wie weiblichen Luer-Kegel bzw. Luer-Lock-Kegel, bei zusammengeschraubten Adaptern flüssigkeitsdicht oder im Wesentlichen flüssigkeitsdicht eingreift oder umgekehrt.

Um die gewünschte Flexibilität des Einsatzes zum Erfassen eines Vials zu erreichen, besteht die Möglichkeit, dass die Umfangswandung des Einsatzes zwischen der Bodenwandung bzw. der Stufe und dem radial nach innen gerichteten Vorsprung zumindest bereichsweise ausgespart ist.

Der hohlzylindrische Außenkörper und der in diesem vorhandene Einsatz sind insbesondere als Spritzgussteile ausgebildet.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung eines der Zeichnung zu entnehmenden bevorzugten Ausführungsbeispiels.

Es zeigen:
- Fig. 1: eine aus zwei Adaptern zusammengebaute Transfer-Mischvorrichtung,
- Fig. 2: die Transfer-Mischvorrichtung in Fig. 1 im Längsschnitt,
- Fig. 3: einer der Adapter der Misch- und Transfervorrichtung gemäß Fig. 1 und 2,
- Fig. 4: der Adapter gemäß Fig. 3 im Schnitt,
- Fig. 5: der andere Adapter der Misch- und Transfervorrichtung gemäß der Fig. 1 und 2,
- Fig. 6: der Adapter gemäß Fig. 5 im Schnitt,
- Fig. 7: ein Detail des Adapters gemäß Fig. 6,
- Fig. 8: der zweite Adapter in einer zu dem ersten Adapter gedrehten Stellung, um ein Trennen zu ermöglichen,
- Fig. 9: die voneinander getrennten Adapter und
- Fig. 10: eine weitere Ausführungsform einer Transfer-Mischvorrichtung.

In den Figuren, in denen gleiche Elemente grundsätzlich mit gleichem Bezugszeichen versehen sind, ist eine Misch- und/oder Transfervorrichtung 10 vom prinzipiellen Aufbau her dargestellt. Aus Gründen der Vereinfachung wird die Misch- und/oder Transfervorrichtung 10 nachstehend als Mischvorrichtung 10 bezeichnet.

Wesentliche Elemente der Mischvorrichtung 10 sind zwei Adapter 12, 14, die mittels einer Schraubverbindung miteinander verbunden werden. Die Adapter 12, 14 dienen zur Aufnahme von nicht dargestellten Behältnissen oder Fläschchen, die auch als Vials bezeichnet werden, deren Inhalte miteinander vermischt werden sollen. Hierzu ist insbesondere vorgesehen, dass ein Vial eine medizinische Substanz insbesondere in Form eines Lyophilisat enthält und das andere Vial eine Flüssigkeit. Das Lyophilisat steht unter Unterdruck, so dass dann, wenn zwischen den Vials eine Verbindung hergestellt wird, die Flüssigkeit aus dem einen Vial in das das Lyophilisat enthaltende Vial gesaugt wird, so dass eine Vermischung zur Zurverfügungstellung eines Medikamentes erfolgen kann.

Im Ausführungsbeispiel dient der Adapter 12 zur Aufnahme eines die Flüssigkeit enthaltenden Vials und der Adapter 14 zur Aufnahme des die medizinische Substanz enthaltenden Vials.

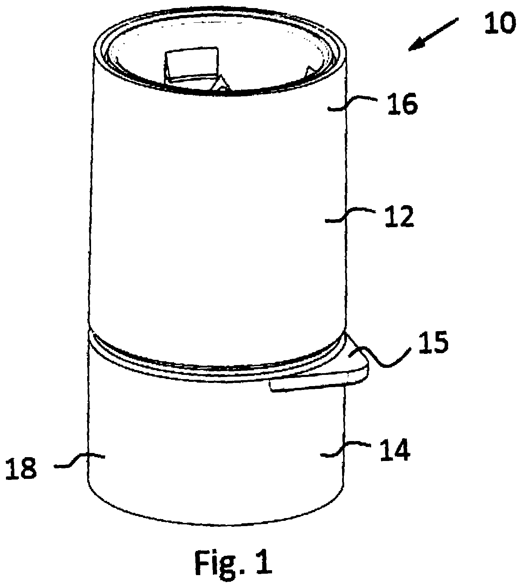

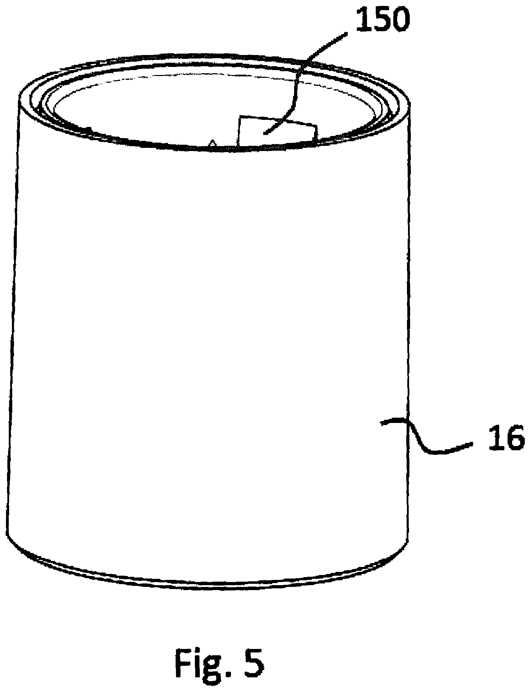

Die Adapter 12, 14 weisen jeweils einen als Gehäuse zu bezeichnenden hohlzylinderförmigen Außenkörper 16, 18 auf, der in der Ausführungsform der Fig. 1 bis 9 umfangsseitig geschlossen und insbesondere aus formstabilem Kunststoff besteht, so dass eine Formbeständigkeit gegeben ist, dass beim Erfassen der Mischvorrichtung 10 diese dem Grunde nach nicht verformbar ist. Hohlzylinder- bzw. hohlkörperförmiger Außenkörper und Gehäuse sind als Synonyme zu bezeichnen, wobei nachstehend aus Gründen der Vereinfachung das in der Zeichnung obere Gehäuse als oberes Gehäuse 16 und das Gehäuse des Adapters 14 als unteres Gehäuse 18 bezeichnet werden.

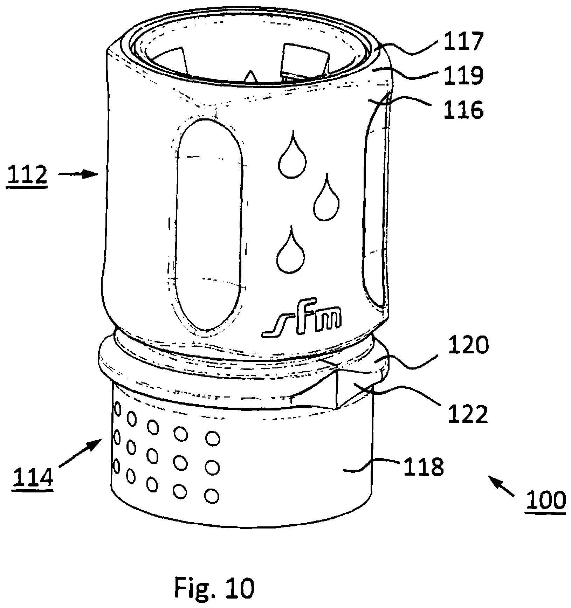

Nach dem Ausführungsbeispiel der Fig. 1 bis 9 weisen die Außenkörper, also die Gehäuse 16, 18 eine hohlzylinderförmige Geometrie auf. Nach dem Ausführungsbeispiel der Fig. 10 weist das Gehäuse 118 des zeichnerisch dargestellten unteren Adapters 114 gleichfalls eine hohlzylinderförmige Geometrie auf, wohingegen der obere Außenkörper 116 des oberen Adapters 112 außenseitig eine mehrkantsäulenförmige wie im Schnitt quadratische Außengeometrie, innenseitig jedoch im Schnitt eine Kreisgeometrie aufweist. Aus Gründen der Vereinfachung wird nachstehend grundsätzlich von hohlzylinderförmiger Geometrie gesprochen, gleichwenn - wie zuvor erläutert - Abweichungen bestehen können, ohne dass die Erfindung verlassen wird. Insoweit ist hohlzylinderförmiger Außenkörper ein Synonym für die in Frage kommenden Hohlkörpergeometrien zu verstehen. Wichtig ist, dass der Außenkörper entsprechend der erfindungsgemäßen Lehre die Funktion erfüllen können, Vials aufzunehmen, und zwar über einen Einsatz, wie nachstehend erläutert wird.

Von dem unteren Gehäuse 18 ragt ein z.B. dreieckförmiger Vorsprung 15 ab, der als Rollschutz bei liegendem Gehäuse 18 dient. Die Form des Vorsprungs 15 ist rein beispielhaft zu verstehen.

Es besteht jedoch auch die Möglichkeit, auf andere Weise einen Rollschutz zu verwirklichen, insbesondere durch die Mehrkantsäulengeometrie gemäß der Fig. 10. Insoweit wird auf die nachstehenden Ausführungen verwiesen.

Nachfolgend wird zunächst das untere Gehäuse 18 beschrieben. Aus der Schnittdarstellung ist erkennbar, dass zwischen den Stirnrändern 20, 22 und senkrecht zur Längsachse des unteren Gehäuses 18 verlaufend eine Zwischenwandung 24 vorhanden ist, von deren Mitte der gleichfalls aus Gründen der Vereinfachung, jedoch nicht schutzeinschränkend nachstehend als weiblicher Luer-Lock-Kegel 26 bezeichnete hohlzylindrische Abschnitt ausgeht, der koaxial von dem oberen Wandabschnitt 28 des unteren Gehäuses 18 umgeben wird und der zwischen der Zwischenwandung 24 und dem in der Zeichnung oberen Stirnrand 20 verläuft.

Über einen Absatz 30 geht der obere Wandabschnitt 28 in einen unteren Wandabschnitt 32 über, der gleichfalls eine Zylindergeometrie aufweist.

Ferner gehen von der Außenfläche des oberen Wandabschnitts 28 ein Außengewinde 34 bzw. Abschnitte eines solchen und somit ein schraubenförmig verlaufender Steg aus, der über Durchtrennungen oder Ausschnitte 36, 38 in dem Wandabschnitt 28 in Abschnitte 40, 42 unterteilt ist.

Die Ausschnitte 36, 38 haben insbesondere herstellungsbedingte Funktion, um ein Entformen des als Spritzgussteil hergestellten Gehäuses 18 zu ermöglichen, da in diesem Bereich von dem weiblichen Luer-Lock-Kegel 26 radial abragende stegförmige Vorsprünge verlaufen, von denen einer mit dem Bezugszeichen 44 gekennzeichnet ist.

In dem zwischen dem unteren Wandabschnitt 32, also der Außenumfangswandung des unteren Gehäuses 18 und der Zwischenwandung 24 umgebenen Bereich des Gehäuses 18 wird erfindungsgemäß ein Einsatz 46 eingebracht, der insbesondere stoffschlüssig mit der Zwischenwandung 24 und vorzugsweise umlaufend verbunden ist. Der Einsatz 46 weist eine koaxial zu dem unteren Wandabschnitt 32 verlaufende Umfangswandung 47 auf, die folglich konzentrisch um die Längsachse des unteren Gehäuses 18 verläuft und als Aufnahme bzw. Halterung des in dem Einsatz 46 zu fixierenden Vials dient. Hierzu ragen von der Umfangswandung 47 des Einsatzes 46 in das Innere des Einsatzes 46 sich erstreckende Vorsprünge 48, 50, 52 ab, die eine hakenförmige Geometrie, also im Schnitt eine Dreiecksgeometrie aufweisen, um nach ordnungsgemäßem Einbringen eines Vials dessen wulstartigen Rand zu hinterfassen und somit ein Fixieren sicherzustellen.

Um eine erforderliche Beweglichkeit der Vorsprünge 48, 50, 52 zu gewährleisten, verläuft zum einen der Einsatz 46, d. h. dessen Umfangswandung 47 zumindest in dem Bereich der Vorsprünge 48, 50, 52, vorzugsweise jedoch vollumfänglich, beabstandet zur Innenseite der Außenumfangswandung 32 des unteren Gehäuses 18. Ferner sind Aussparungen vorgesehen, die sich zwischen den Vorsprüngen 48, 50, 52 und der sich entlang der Zwischenwandung 24 erstreckenden Bodenwandung 54 des Einsatzes 46 erstrecken. Eine Aussparung ist mit dem Bezugszeichen 51 gekennzeichnet.

Die Bodenwandung 54 verläuft erwähntermaßen bei fixiertem Einsatz 46 entlang der Außenseite der Zwischenwandung 24 des unteren Gehäuses 18, wobei zwischen der Außenseite der Bodenwandung 54 und der Außenseite der Zwischenwandung 24 ein flächiges Filter 56 vorgesehen ist. Dieses kann zuvor an der Außenseite der Bodenwandung 54 des Einsatzes 46 fixiert worden sein, bevor der Einsatz 46 in das untere Gehäuseteil 32 eingesetzt und mit diesem verbunden wird.

Um ein ordnungsgemäßes Positionieren des Einsatzes 46 sicherzustellen, weist die Bodenwandung 54 einen über dessen Außenseite vorstehenden umlaufenden und auf einem Kreis liegenden Steg 58 auf, der in eine entsprechende Aussparung 60 in der Außenseite der Zwischenwandung 24 einsetzbar ist, um somit eine Zentrierung zu ermöglichen. Andere Zentriermaßnahmen sind gleichfalls möglich und werden von der Erfindung erfasst. Entsprechende Zentrierungen schließen auch punktuell angeordnete Zentriermittel ein.

Des Weiteren kann der Einsatz 46 im Bereich der Innenseite des Absatzes 30 des unteren Gehäuses 18 eine Stufe 62 aufweisen, wodurch zusätzlich das gewünschte koaxiale Ausrichten des Einsatzes 46 zu dem unteren Gehäuse 18 gewährleistet ist.

Im Bereich des stegartigen Vorsprungs 58 sollte der Einsatz 46 mit der Zwischenwandung 24 insbesondere mittels Ultraschallschweißens stoffschlüssig verbunden sein, wodurch eine Flüssigkeitsdichtheit zwischen dem Einsatz 46 und der Zwischenwandung 24 gewährleistet ist.

Von der Bodenwandung 54 erstreckt sich entgegengesetzt zu dem weiblichen Luer-Lock-Kegel 26 verlaufend ein einen Hohlnadelkörper bzw. Kanülenkörper bildendes Kunststoffspike 64 mit einer Spitze 66, die beim Einsetzen des Vials in den Einsatz 46 dessen Verschluss durchsetzt, damit in nachstehend beschriebener Art eine Verbindung über den weiblichen Luer-Lock-Kegel 26 zu dem von dem oberen Gehäuse 16 der Mischvorrichtung 12 aufgenommenen Vial hergestellt werden kann.

Wie sich insbesondere aus der Schnittdarstellung ergibt, weist der Querschnitt, also das Lumen des Spikes 64 in der Durchtrittsöffnung durch die Bodenwandung 54 und die Zwischenwandung 24 einen kleineren Querschnitt als der zwischenwand- und somit bodenwandseitig verlaufende Luer-Lock-Kegel 26 auf.

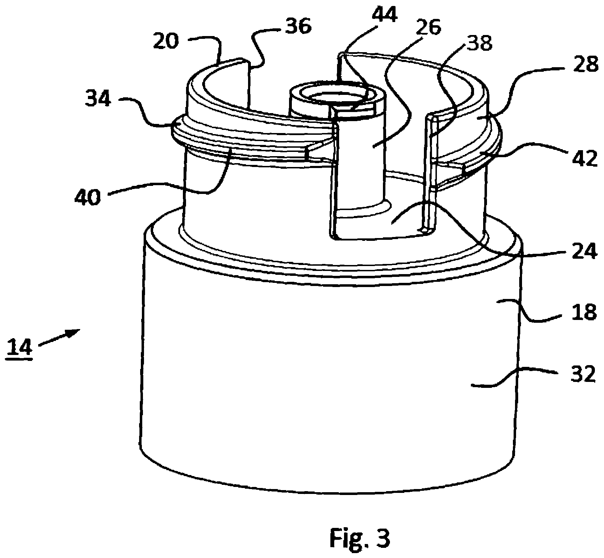

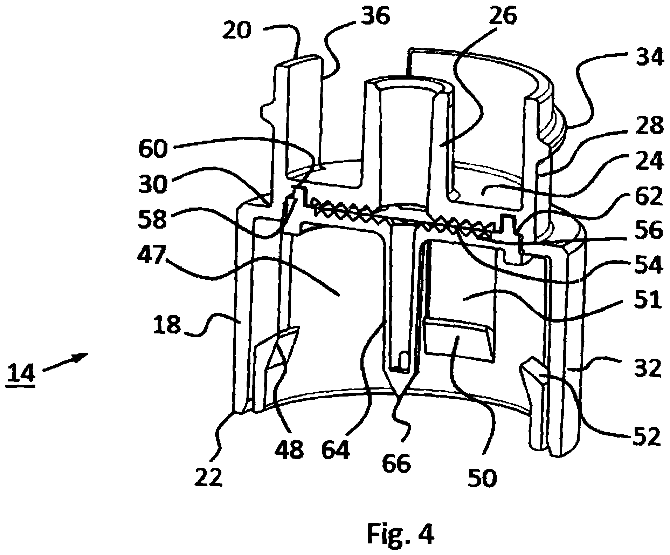

Entsprechend den Erläuterungen im Zusammenhang mit den Fig. 3 und 4 ist in das obere Gehäuse 16 ein Einsatz 146 einbringbar, der koaxial von der Außenwandung 132 des oberen Gehäuses 16 umgeben und insbesondere umfangsseitig vollständig beabstandet zu deren Innenseite verläuft. Auch der Einsatz 146 weist eine Bodenwandung 154 auf, von dem ein die Funktion eines Kanülenkörpers aufweisender Kunststoffspike 164 mit Spitze 166 abragt und sich entlang der Längsachse der Mischvorrichtung 10 und damit des oberen Gehäuses 16 erstreckt. Der Spike 164 wird folglich von der Umfangswandung 147 des Einsatzes 146 koaxial umgeben.

In der Umfangswandung 147 sind Aussparungen 151 vorhanden, die von radial ins Innere des Einsatzes 146 ragenden hakenförmigen Vorsprüngen 148, 150, 152 begrenzt werden, die entsprechend zuvor erfolgter Erläuterungen zum Hinterfassen des Randes eines von dem Einsatz 146 aufzunehmenden Vials dienen. Insoweit wird auf die zuvor erfolgten Erläuterungen verwiesen.

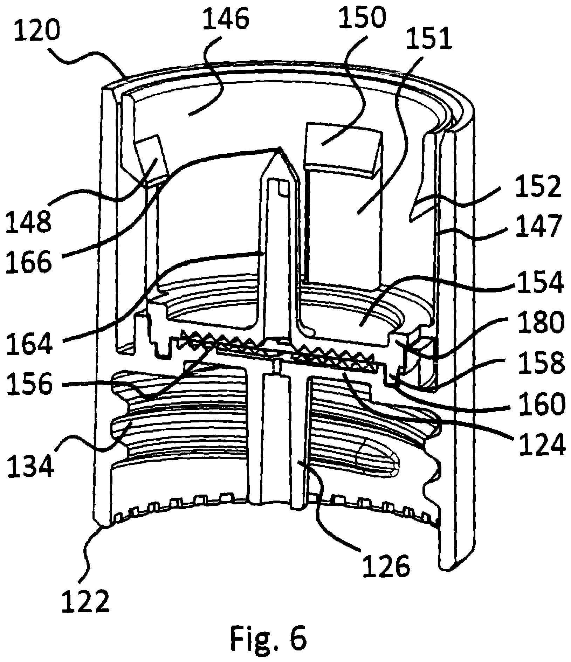

Entsprechend der Fig. 6 geht von der Bodenwandung 154 des Einsatzes 146 ein sich entlang eines Kreises erstreckender axial verlaufender stegförmiger Vorsprung 158 aus, der in eine entsprechende Aussparung wie Nut 160 in der zwischen den Stirnrändern 122, 120 des oberen Gehäuses 16 verlaufenden Zwischenwandung 124 eingreift.

Von der Zwischenwandung 124 und sich entlang der Längsachse des oberen Gehäuses 16 erstreckend geht ein hohlzylindrischer Abschnitt 126 wie Luer-Kegel aus, der bei zusammengesetzten Adaptern 12, 14, also Gehäusen 16, 18 in den weiblichen Luer-Lock-Kegel 26 eingreift. Nachstehend wird der hohlzylindrische Abschnitt 126 aus Gründen der Einfachheit als Luer-Kegel bezeichnet, ohne dass hierdurch eine Einschränkung der Erfindung erfolgen soll.

Entlang der Außenseite der Bodenwandung 154 des Einsatzes 146 verläuft ein Filter 156, das vor dem stoffschlüssigen Verbinden wie Verschweißen des Einsatzes 146 mit der Zwischenwandung 124 mit der Außenseite verbunden wird. Allerdings besteht auch die Möglichkeit, den Adapter 12 ohne Filter 156 herzustellen. Der Adapter 14 sollte demgegenüber ein Filter 56 aufweisen.

Abweichend von dem Einsatz 46 des unteren Gehäuses 18 geht die Bodenwandung 154 des Einsatzes 146 über eine Stufe 180 in die Umfangswandung 147 über, so dass sich der Einsatz 146 dem Grunde nach aus zwei zylindrischen Abschnitten, nämlich dem von der Bodenwandung 154 begrenzten und dem äußeren hohlzylindrischen Abschnitt größeren Querschnitts zusammensetzt, von dem die Vorsprünge 146, 150 radial nach innen abragen.

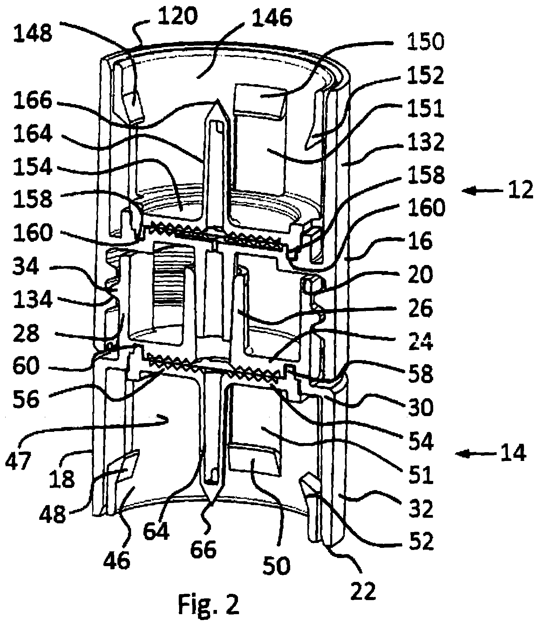

Des Weiteren verläuft zwischen der Zwischenwandung 124 und der dem unteren Gehäuse 18 zugewandten Stirnwand 122 eine ein Innengewinde 134 bildende nutförmige Vertiefung, die im Ausführungsbeispiel aus zwei Gängen besteht, wie die Schnittdarstellung der Fig. 2 verdeutlicht. Die Anzahl der Gänge beschränkt jedoch nicht die Erfindung. Vielmehr greift in das Innengewinde 134 das Außengewinde 34 des unteren Gehäuses 18 dann ein, wenn die Gehäuse 16, 18, also die Adapter 12, 14 miteinander verbunden werden sollen.

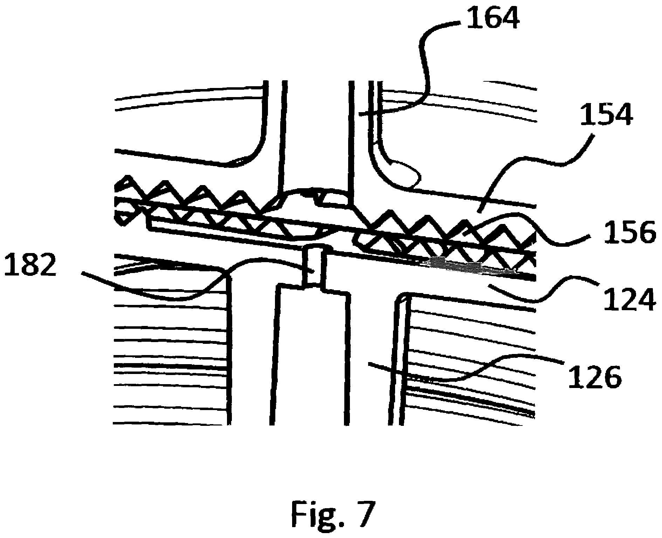

Aus der Detaildarstellung der Fig. 7 wird insbesondere erkennbar, dass zwischen der Zwischenwandung 124 des oberen Gehäuses 16 und der Bodenwandung 154 des Einsatzes 146 das Filter 156 verläuft.

Aus der Detaildarstellung wird insbesondere auch klar erkennbar, dass der bodenseitig verlaufende Querschnitt des von der Zwischenwandung 124 ausgehenden einen Hohlnadel- bzw. Kanülenkörper bildenden Spikes 164 größer als die Durchtrittsöffnung 182 des Luer-Kegels 126 durch die Zwischenwandung 124 ist, wodurch eine Blende zwischen dem Spike 164 und dem Luer-Kegel 126 gebildet wird, die zur Drosselung der durch den Spike 164, den Luer-Kegel 126 und über den Spike 64 zu dem von diesem durchstochenen und das Lyophilisat enthaltenden Vial strömenden Flüssigkeit dient.

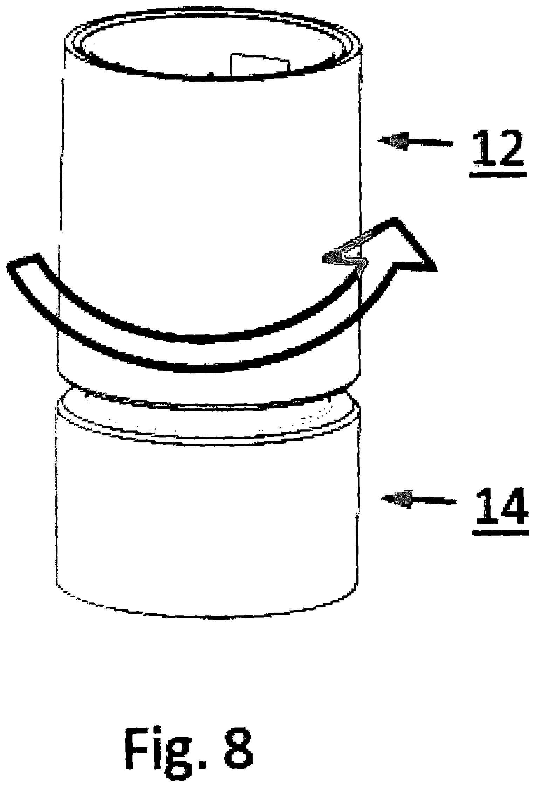

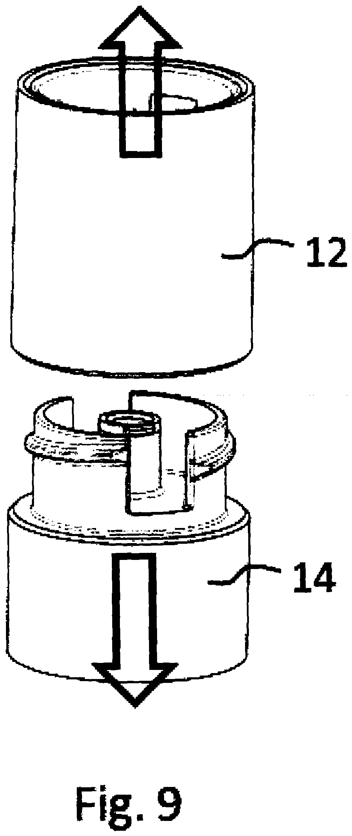

Das Innen- und Außengewinde 34, 134 sind entsprechend der Darstellung der Fig. 8 und 9 derart ausgebildet, dass nach einer etwa 180°-Drehung zwischen oberem und unterem Adapter 12, 14 zueinander ein Lösen dieser voneinander möglich ist bzw. ein Verbinden miteinander erfolgt, wobei der Luer-Kegel 126 derart in den weiblichen Luer-Lock-Kegel 26 eindringt, dass eine hinreichende Abdichtung gegeneinander erfolgt und somit die Flüssigkeit von einem Vial in das andere Vial strömen kann.

Unabhängig hiervon sind die Fig. selbsterklärend und geben die die Erfindung prägenden Merkmale im hinreichenden Umfang wieder.

Erwähntermaßen unterscheidet sich die Transfervorrichtung 100 nach der Fig. 10 von der der Fig. 1 bis 9 ausschließlich durch die äußere Form und nicht durch die erfindungsgemäß als Einsätze ausgebildeten Aufnahmen für die Vials. Das obere Gehäuse 116 kann im Zwei-Komponenten-Kunststoff-Spritzgiessverfahren hergestellt sein. Das obere Gehäuse 116 besteht dabei aus einem inneren eine Hohlzylindergeometrie aufweisenden Basiskörper 117, das umfangseitig mit einem thermoplastischen Elastomer partiell umspritzt ist (Außenschicht 119). Diese Bereiche verleihen dem umspritzten oberen Gehäuse 116 eine Außengeometrie einer Mehrkantsäule. Hierdurch ergibt sich eine bessere Haptik, da die Transfervorrichtung 100 besser in der Hand liegt, griffiger und weicher wird. Da die zu mischenden Medien kühl gelagert werden und mit diesen die Transfervorrichtung 114 in einer Packung bereitgestellt wird, ergibt sich der Vorteil, dass aufgrund der thermoplastischen Umhüllung (Außenschicht 119) das obere Gehäuse 116 beim Anfassen "wärmer" empfunden wird.

Die in der Fig. 10 eingezeichneten Aussparungen sind Freiräume in der Außenschicht 119, durch die der Basiskörper 117 erfassbar ist.

Des Weiteren erkennt man aus der Fig. 10, dass das untere Gehäuse 118 in seinem oberen Randbereich einen umlaufenden abragenden Rand 120 aufweist, durch den ein Erfassen des unteren Gehäuses 118 beim Zusammenschrauben mit dem oberen Gehäuse 116 erleichtert werden soll. Hierzu weist der Rand 120 zusätzlich diametral gegenüberliegende Abflachungen 122 bzw. im Schnitt V-förmige Vertiefungen auf. Eine dieser ist in Fig. 10 dargestellt.

Das untere Gehäuse 118 besteht insbesondere aus einem Acrylpolymer. Das obere Gehäuse 116 sowie die Einsätze 46, 146 können aus Methylmetacrylat Acrylnitril Butatien und Styrol (MABS) bestehen.

Die Transfervorrichtung selbst kann eine Höhe von z. B. 46 mm bei einem Durchmesser von 30 mm aufweisen. Die Wandstärke des unteren Gehäuses 18, 118 und des oberen Gehäuses 16 bzw. Basiskörpers 117 sollte im Bereich zwischen 1,2 mm und 1,6 mm liegen.

## Patentansprüche

1. Misch- und/oder Transfervorrichtung (10, 100) zum Mischen bzw. Überleiten einer ersten Substanz und einer zweiten Substanz, die in einem ersten bzw. zweiten Behältnis vorhanden sind, umfassend
- einen ersten Adapter (14, 114) mit einer zur Aufnahme des ersten Behältnisses geeigneten Umfangswandung (47), entlang der und von dieser zumindest abschnittsweise umgeben ein Kanülenkörper (64) verläuft, sowie einem mit dem Kanülenkörper in Verbindung stehenden hohlzylindrischen Abschnitt (26),
- einen zweiten Adapter (12, 112) mit einer zur Aufnahme des zweiten Behältnisses geeigneten Umfangswandung (147), entlang der und koaxial von dieser zumindest abschnittsweise umgeben ein Kanülenkörper (164) verläuft, sowie einem mit dem Kanülenkörper in Verbindung stehenden hohlzylindrischen Abschnitt (126),
wobei bei zusammengesetztem ersten und zweiten Adapter die hohlzylindrischen Abschnitte vorzugsweise flüssigkeitsabdichtend ineinander greifen und der erste und der zweite Adapter miteinander lösbar schraubbar verbunden sind,
**dadurch gekennzeichnet,**
**dass** der erste und der zweite Adapter (12, 14, 112, 114) jeweils einen hohlzylinderförmigen Außenkörper (16, 18, 116, 118) mit Außenumfangswandung (32, 132) und eine senkrecht zur Längsachse des Außenkörpers verlaufende Zwischenwandung (24, 124) aufweist,
**dass** bei zusammengesetzten Adaptern ein ein Innengewinde (134) aufweisender Abschnitt der Außenumfangswandung des einen Adapters einen ein Außengewinde (34) aufweisenden Abschnitt (28) der Außenumfangswandung des anderen Adapters bei ineinander greifenden Gewindeabschnitten umgibt und dass der hohlzylinderförmige Außenkörper des ersten und/oder des zweiten Adapters einen Einsatz (46, 146) mit der das erste oder zweite Behältnis aufnehmenden Umfangswandung (47, 147) aufweist.

2. Misch- und/oder Transfervorrichtung nach Anspruch 1,
**dadurch gekennzeichnet,**
**dass** jeder Adapter (12, 14) einen Einsatz (46, 146) mit der das erste bzw. zweite Behältnis aufnehmenden Umfangswandung (47, 147) aufweist.

3. Misch- und/oder Transfervorrichtung nach Anspruch 1 oder 2,
**dadurch gekennzeichnet,**
**dass** die insbesondere konzentrisch von der Außenumfangswandung (32, 132) des Außenkörpers (16, 18), wie Gehäuse, umgebene Umfangswandung (47, 147) des Einsatzes (46, 146) radial sich ins Innere des Einsatzes erstreckenden Vorsprünge (48, 50, 52, 148, 150, 152) zum Halten des Behältnisses aufweist, wobei die Umfangswandung zumindest im Bereich der Vorsprünge beabstandet zur Innenseite der Außenumfangswandung verläuft.

4. Misch- und/oder Transfervorrichtung nach zumindest einem der vorhergehenden Ansprüche,
**dadurch gekennzeichnet,**
**dass** der Einsatz (46, 146) eine Bodenwandung (54, 154) aufweist, die bei mit dem hohlzylinderförmigen Außenkörper (16, 18) verbundenem Einsatz, gegebenenfalls über ein Filterelement (56, 156), auf der Zwischenwandung (24, 124) aufliegt.

5. Misch- und/oder Transfervorrichtung nach zumindest einem der vorhergehenden Ansprüche,
**dadurch gekennzeichnet,**
**dass** von der Bodenwandung (54, 154) des Einsatzes (46, 146) ein Zentrierelement (58, 158) abragt, das in eine angepasste Aufnahme (60, 160) in der Zwischenwandung (24, 124) bei mit dem hohlzylinderförmigen Außenkörper (16, 18) verbundenem Einsatz eingreift oder umgekehrt, wobei insbesondere das Zentrierelement (58, 158) ein ringförmig verlaufendes Stegelement ist, das in eine ringförmig verlaufende Aufnahme (60, 160) wie Nut eingreift.

6. Misch- und/oder Transfervorrichtung nach zumindest einem der vorhergehenden Ansprüche,
**dadurch gekennzeichnet,**
**dass** von der Bodenwandung (54, 154) des Einsatzes (46, 146) der Kanülenkörper (64, 164) und/oder von der Zwischenwandung (24, 124) des hohlzylinderförmigen Außenkörpers (16, 18) der hohlzylinderförmige Abschnitt (26, 126) ausgeht bzw. ausgehen.

7. Misch- und/oder Transfervorrichtung nach zumindest einem der vorhergehenden Ansprüche,
**dadurch gekennzeichnet,**
**dass** zumindest in einem der Adapter (12, 14) der wirksame Querschnitt des Kanülenkörpers (164) in der Bodenwandung (154) größer ist als Querschnitt einer Verbindungsöffnung (182) zu dem von der Zwischenwandung (124) ausgehenden hohlzylinderförmigen Abschnitt (126).

8. Misch- und/oder Transfervorrichtung nach zumindest einem der vorhergehenden Ansprüche,
**dadurch gekennzeichnet,**
**dass** bei zusammengeschraubten Adaptern (12, 14) deren vorzugsweise geschlossenen Außenflächen - ggfs. mit Ausnahme eines zwischen diesen verlaufenden Spalts - bündig ineinander übergehen.

9. Misch- oder Transfervorrichtung nach zumindest einem der vorhergehenden Ansprüche,
**dadurch gekennzeichnet,**
**dass** Außenseite der Bodenwandung (54, 154) des Einsatzes (46, 146) strukturiert ist, wobei die Bodenwandung ggfs. von einem flächigen Filterelement als das Filter (56, 156) abgedeckt ist.

10. Misch- und/oder Transfervorrichtung nach zumindest einem der vorhergehenden Ansprüche,
**dadurch gekennzeichnet,**
**dass** der Einsatz (46, 146) einen bodenseitig verlaufenden hohlzylindrischen ersten Abschnitt und einen über eine Stufe (180) übergehenden und die Umfangswandung (147) bildenden zweiten Abschnitt größeren Außendurchmessers aufweist, und dass die Stufe auf einem von der Zwischenwandung (124) des hohlzylinderförmigen Außenkörpers (12) axial abragenden Vorsprung abgestützt ist.

11. Misch- und/oder Transfervorrichtung nach zumindest einem der vorhergehenden Ansprüche,
**dadurch gekennzeichnet,**
**dass** der Einsatz (46, 146) mit dem hohlzylinderförmigen Außenkörper (16, 18) umlaufend luft- oder flüssigkeitsdicht verbunden ist, insbesondere mit dem hohlzylindrischen Außenkörper (16, 18) bodenseitig, insbesondere mittels Ultraschallschweißens, stoffschlüssig verbunden ist.

12. Misch- und/oder Transfervorrichtung nach zumindest einem der vorhergehenden Ansprüche,
**dadurch gekennzeichnet,**
**dass** von der Zwischenwandung (124) des das Innengewinde (134) aufweisenden hohlzylinderförmigen Außenkörpers (16) ein männlicher hohlzylindrischer Abschnitt (126), wie Luer-Kegel, abragt, der in einen von der Zwischenwandung (24) des das Außengewinde (34) aufweisenden hohlkörperförmigen Außenkörpers (18) abragenden weiblichen hohlzylindrischen Abschnitt (26), wie weiblichen Luer-Lock-Kegel, bei zusammengeschraubten Adaptern (12, 14) flüssigkeitsdicht oder im Wesentlichen flüssigkeitsdicht eingreift oder umgekehrt.

13. Misch- und/oder Transfervorrichtung nach zumindest einem der Ansprüche 4 bis 14,
**dadurch gekennzeichnet,**
**dass** die Umfangswandung (47, 147) des Einsatzes (46, 146) zwischen der Bodenwandung (54, 156) bzw. der Stufe (180) und dem radial nach innen gerichteten Vorsprung (48, 50, 52, 148, 150) zumindest bereichsweise ausgespart ist, und/oder dass die Umfangswandung (47, 147) des Einsatzes (46, 146) zumindest abschnittsweise, vorzugsweise vollständig, beabstandet zur Innenfläche der Außenumfangswandung verläuft.

14. Misch- und/oder Transfervorrichtung nach zumindest einem der vorhergehenden Ansprüche,
**dadurch gekennzeichnet,**
**dass** der hohlzylindrische Außenkörper (16, 18) und/oder der Einsatz (46, 146) ein Spritzgussteil sind bzw. ist, wobei vorzugsweise zumindest einer der hohlzylinderförmigen Außenkörper (116) der Adapter (12) in Zwei-Komponenten-Spritzgießverfahren hergestellt ist und vorzugsweise der zumindest eine hohlzylinderförmige Außenkörper (116) aus einem inneren eine Hohlzylindergeometrie aufweisenden Basiskörper (117) aufweist, der teilweise umspritzt ist, insbesondere zur Bildung einer Mehrkantsäulen-Außengeometrie.

15. Misch- und/oder Transfervorrichtung nach zumindest einem der vorhergehenden Ansprüche,
**dadurch gekennzeichnet,**
**dass** zumindest einer der hohlzylinderförmigen Außenkörper (116) innenseitig eine Zylindergeometrie und außenseitig eine von einer Zylindergeometrie abweichende Geometrie, insbesondere Mehrkantsäulengeometrie, aufweist.

## Claims

1. A mixing and/or transferring device (10, 100) for mixing or transferring a first substance and a second substance that are present in a first and a second receptacle, respectively comprising
- a first adapter (14, 114) with a circumferential wall (47), which is suitable for accepting the first receptacle, along said circumferential wall extends and which at least in sections surrounds a cannula body (64), as well as with a hollow-cylindrical section (26) that is connected to the cannula body,
- a second adapter (12, 112) with a circumferential wall (147), which is suitable to accept the second receptacle, along said circumferential wall extends and which a least in sections coaxially surrounds a cannula body (164), as well as with a hollow-cylindrical section (126) that is connected to the cannula body,
whereby when the first adapter and the second adapter have been assembled, the hollow-cylindrical sections preferably inter-engage in a liquid-tight manner and the first adapter and the second adapter are connected in a detachable and screwable manner, **characterized in**
**that** each of the first and the second adapters (12, 14, 112, 114) comprises a hollow-cylindrical outer body (16, 18, 116, 118) with an exterior circumferential wall (32, 132) and, extending normal to the longitudinal axis of the outer body, a partition wall (24, 124), in that, when the adapters have been assembled, a section (128), which has an inner thread (134), of the outer circumferential wall of the one adapter surrounds a section (28), which has an external thread (34), of the outer circumferential wall of the other adapter, with inter-engaging threaded sections, and that the hollow-cylindrical outer body of the first and/or second adapter comprises an insert (46, 146) with a circumferential wall (47, 147) that accepts the first or the second receptacle.

2. The mixing and/or transferring device of claim 1,
**characterized in**
**that** each adapter (12, 14) comprises an insert (46, 146) with a circumferential wall (47, 147) that accepts the first or the second receptacle.

3. The mixing and/or transferring device of claim 1 or 2,
**characterized in**
**that** the circumferential wall (47, 147) of the insert (46, 146), which is in particular concentrically surrounded by the outer circumferential wall (32, 132) of the outer body (16, 18), such as a housing, comprises projections (48, 50, 52, 148, 150, 152) that radially extend into the interior of the insert for holding the receptacle, whereby the circumferential wall at least in the area of the projections extends spaced-apart relative to the interior side of the outer circumferential wall.

4. The mixing and/or transferring device of at least one of the preceding claims,
**characterized in**
**that** the insert (46, 146) comprises a bottom wall (54, 154), which, when the insert is connected with the hollow-cylindrical outer body (16, 18), is supported on the partition wall (24, 124), possibly via a filter element (56, 156).

5. The mixing and/or transferring device of at least one of the preceding claims,
**characterized in**
**that** from the bottom wall (54,154) of the insert (46,146) protrudes, a centering element (58, 158), which, when the hollow-cylindrical outer body (16, 18) is connected to the insert, engages into an adapted acceptance (60, 160) in the partition wall (24, 124) or vice versa, wherein the centering element (58, 158) is embodied as an annularly extending ledge element, which engages into an annularly extending recess (60, 160), such as a groove.

6. The mixing and/or transferring device of at least one of the preceding claims,
**characterized in**
**that** from the bottom wall (54, 154) of the insert (46, 146) originates the cannula body (64,164) and/or from the partition wall (24,124) of the hollow-cylindrical outer body (16, 18) originates the hollow-cylindrical section (26,126).

7. The mixing and/or transferring device of at least one of the preceding claims,
**characterized in**
**that** in at least one of the adapters (12, 14), the effective cross section of the cannula body (164) in the bottom wall (154) is greater than the cross-section of a connecting opening (182) leading to the hollow-cylindrical section (126) that originates from the partition wall (124).

8. The mixing and/or transferring device of at least one of the preceding claims,
**characterized in**
**that** when the adapters (12, 14) have been screwed together, their preferably solid external surfaces should form a flush transitional surface - possibly with the exception of a gap extending between them.

9. The mixing and/or transferring device of at least one of the preceding claims, **characterized in**
**that** the exterior side of the bottom wall (54, 154) of the insert (46, 146) is structured, whereby the bottom wall possibly is covered by a planar filter element as the filter (56, 156).

10. The mixing and/or transferring device of at least one of the preceding claims,
**characterized in**
**that** the insert (46, 146) comprises a hollow-cylindrical first section extending on the bottom side and, via a step (180) as transition, a second section of greater external diameter that forms the circumferential wall (147), and in that the step is supported on a projection that axially protrudes from the partition wall (124) of the hollow-cylindrical outer body (12).

11. The mixing and/or transferring device of at least one of the preceding claims,
**characterized in**
**that** the insert (46, 146) is circumferentially connected to the hollow-cylindrical outer body (16, 18) in an airtight or liquid-tight manner, preferably on the bottom side in a materially bonded manner to the hollow-cylindrical outer body (16, 18), in particular by means of ultrasonic welding.

12. The mixing and/or transferring device of at least one of the preceding claims.
**characterized in**
**that** from the partition wall (124) of the hollow-cylindrical outer body (16) that possesses the internal thread (134) protrudes a male hollow-cylindrical section (126), such as a Luer cone, which engages liquid-tight or essentially liquid-tight with a female hollow-cylindrical section (26), such as a female Luer Lock cone, which, protrudes from the partition wall (24) of the external-thread-equipped (34) outer body (18), when the adapters (12, 14) have been screwed together, or vice versa.

13. The mixing and/or transferring device of claims 4 to 14,
**characterized in**
**that** the circumferential wall (47, 147) of the insert (46, 146) is recessed in at least some areas between the bottom wall (54, 156), or the step (180), and the radially inward protruding projection (48, 50, 52, 148, 150) and/or the circumferential wall (47, 147) of the insert (46, 146) extends, at least in sections but preferably entirely, spaced-apart relative to the interior surface of the outer circumferential wall.

14. The mixing and/or transferring device of at least one of the preceding claims,
**characterized in**
**that** the hollow-cylindrical outer body (16,18) and/or the insert (46, 146) are/is embodied as an injection-molded part, wherein preferably the at least one of the hollow-cylindrical outer bodies (116) of the adapters (12) is manufactured in a two-component injection-molding process and preferably the at least one hollow-cylindrical outer body (116) consists of an inner base body (117), which possesses a hollow-cylindrical geometry and which is partially over-molded, in particular to create a polygonal-column external geometry.

15. The mixing and/or transferring device of at least one of the preceding claims,
**characterized in**
**that** at least one of the hollow-cylindrical outer bodies (116) possesses on the inside a cylindrical geometry and on the outside a geometry different from a cylindrical geometry, in particular a polygonal-column geometry.

## Revendications

1. Dispositif de mélange et/ou de transfert (10, 100) pour mélanger ou transférer une première substance et une seconde substance, se trouvant respectivement dans un premier et un second récipient, comprenant
- un premier adaptateur (14, 114) avec une paroi périphérique (47) appropriée à recevoir le premier récipient, le long de laquelle s'étend un corps de canule (64) entouré au moins par section par ladite paroi, ainsi qu'avec une section cylindrique creuse (26) reliée au corps de canule,
- un second adaptateur (12, 112) avec une paroi périphérique (147) appropriée à recevoir le second récipient, le long de laquelle s'étend un corps de canule (164) entouré coaxialement au moins par section par ladite paroi, ainsi qu'avec une section cylindrique creuse (126) reliée au corps de canule,
sachant que lors de l'assemblage du premier et du second adaptateurs, les sections cylindriques creuses s'emboîtent les unes dans les autres, de préférence en étant étanches aux liquides et que le premier et second adaptateurs sont vissés ensemble de manière amovible,
**caractérisé en ce**
**que** le premier et le second adaptateurs (12, 14, 112, 114) comprennent respectivement un corps extérieur cylindrique creux (16, 18, 116, 118) avec une paroi périphérique extérieure (32, 132) et une paroi intermédiaire (24, 124) s'étendant perpendiculairement à l'axe longitudinal du corps extérieur,
**que**, lorsque les adaptateurs sont assemblés, une section à filetage intérieur (134) de la paroi périphérique extérieure de l'un des adaptateurs entoure une section (28) à filetage extérieur (34) de la paroi périphérique extérieure de l'autre adaptateur lorsque les sections filetées sont reliées l'une à l'autre et que le corps extérieur cylindrique creux du premier et/ou du second adaptateur comprend un insert (46, 146) avec la paroi périphérique (47, 147) recevant le premier ou le second récipient.

2. Dispositif de mélange et/ou de transfert selon la revendication 1,
**caractérisé en ce**
**que** chaque adaptateur (12, 14) comprend un insert (46, 146) dont la paroi périphérique (47, 147) reçoit le premier ou le second récipient.

3. Dispositif de mélange et/ou de transfert selon la revendication 1 ou 2,
**caractérisé en ce**
**que** la paroi périphérique (47, 147) de l'insert (46, 146), entourée en particulier de manière concentrique par la paroi périphérique extérieure (32, 132) du corps extérieur (16, 18), tel un boîtier, comprend des saillies (48, 50, 52, 148, 150, 152) s'étendant radialement vers l'intérieur de l'insert pour maintenir le récipient, sachant que, au moins dans la zone des saillies, la paroi périphérique s'étend à distance du côté intérieur de la paroi périphérique extérieure.

4. Dispositif de mélange et/ou de transfert selon au moins l'une des revendications précédentes,
**caractérisé en ce**
**que** l'insert (46, 146) comprend une paroi de fond (54, 154) qui, lorsque l'insert est relié au corps extérieur cylindrique creux (16, 18), repose sur la paroi intermédiaire (24, 124), le cas échéant via un élément filtrant (56, 156).

5. Dispositif de mélange et/ou de transfert selon au moins l'une des revendications précédentes,
**caractérisé en ce**
**qu'**un élément de centrage (58, 158) dépasse de la paroi de fond (54, 154) de l'insert (46, 146), prend prise dans un logement adapté (60, 160) dans la paroi intermédiaire (24, 124) lorsque l'insert est relié au corps extérieur cylindrique creux (16, 18) ou inversement, sachant en particulier que l'élément de centrage (58, 158) est un élément nervuré annulaire qui prend prise dans un logement annulaire (60, 160) tel qu'une rainure.

6. Dispositif de mélange et/ou de transfert selon au moins l'une des revendications précédentes,
**caractérisé en ce**
**que** la section cylindrique creuse (26, 126) sort de la paroi de fond (54, 154) de l'insert (46, 146) des corps de canule (64, 164) et/ou de la paroi intermédiaire (24, 124) du corps extérieur cylindrique creux (16, 18).

7. Dispositif de mélange et/ou de transfert selon au moins l'une des revendications précédentes,
**caractérisé en ce**
**que**, dans au moins l'un des adaptateurs (12, 14), la section transversale efficace du corps de canule (164) de la paroi de fond (154) est supérieure à la section d'une ouverture de communication (182) par rapport à la section cylindrique creuse (126) sortant de la paroi intermédiaire (124).

8. Dispositif de mélange et/ou de transfert selon au moins l'une des revendications précédentes,
**caractérisé en ce**
**que**, lorsque les adaptateurs (12, 14) sont vissés, leurs surfaces extérieures de préférence fermées - éventuellement à l'exception d'une fente s'entendant entre ces dernières - s'emboîtent par affleurement les unes dans les autres.

9. Dispositif de mélange ou de transfert selon au moins l'une des revendications précédentes,
**caractérisé en ce**
**que** le côté extérieur de la paroi de fond (54, 154) de l'insert (46, 146) est structuré, sachant que la paroi de fond est éventuellement recouverte d'un élément filtrant plat tel qu'un filtre (56, 156).

10. Dispositif de mélange et/ou de transfert selon au moins l'une des revendications précédentes,
**caractérisé en ce**
**que** l'insert (46, 146) comprend une première section cylindrique creuse s'étendant côté fond et une seconde section d'un plus grand diamètre extérieur se propageant au-dessus d'une marche (180) et formant la paroi périphérique (147), et que la marche est appuyée sur une saillie dépassant axialement de la paroi intermédiaire (124) du corps extérieur cylindrique creux (12).

11. Dispositif de mélange et/ou de transfert selon au moins l'une des revendications précédentes,
**caractérisé en ce**
**que** l'insert (46, 146) est relié en périphérie au corps extérieur cylindrique creux (16, 18) en étant étanche à l'air ou aux liquides, notamment au corps extérieur cylindrique creux (16, 18) côté fond, en particulier par liaison de matière par soudage par ultrasons.

12. Dispositif de mélange et/ou de transfert selon au moins l'une des revendications précédentes,
**caractérisé en ce**
**qu'**une section cylindrique creuse mâle (126), telle qu'un cône Luer, dépasse de la paroi intermédiaire (124) du corps extérieur cylindrique creux (16) comprenant le filetage intérieur (134), ladite section prenant prise dans une section cylindrique creuse femelle (26), telle qu'un cône femelle Luer Lock, dépassant de la paroi intermédiaire (24) du corps extérieur en forme de corps creux (18) comprenant le filetage extérieur (34), lorsque les adaptateurs (12, 14) sont vissés ensemble en étant étanche aux liquides ou sensiblement étanche aux liquides ou inversement.

13. Dispositif de mélange et/ou de transfert selon au moins l'une des revendications 4 à 14,
**caractérisé en ce**
**que** la paroi périphérique (47, 147) de l'insert (46, 146) est au moins partiellement évidée entre la paroi de fond (54, 156) ou la marche (180) et la saillie (48, 50, 52, 148, 150) dirigée radialement vers l'intérieur, et/ou que la paroi périphérique (47, 147) de l'insert (46, 146) s'étend au moins par section, de préférence entièrement, à distance de la surface intérieure de la paroi périphérique extérieure.

14. Dispositif de mélange et/ou de transfert selon au moins l'une des revendications précédentes,
**caractérisé en ce**
**que** le corps extérieur cylindrique creux (16, 18) et/ou l'insert (46, 146) est/sont une pièce moulée par injection, sachant que de préférence au moins l'un des corps extérieurs cylindriques creux (116) des adaptateurs (12) est fabriqué par processus d'injection bi-composants et que l'au moins un corps extérieur cylindrique creux (116) présente de préférence un corps de base intérieur (117) présentant une géométrie cylindrique creuse, lequel est partiellement extrudé, en particulier pour la formation d'une géométrie extérieure en forme de colonne polygonale.

15. Dispositif de mélange et/ou de transfert selon au moins l'une des revendications précédentes,
**caractérisé en ce**
**qu'**au moins l'un des corps extérieurs cylindriques creux (116) comprend à l'intérieur une géométrie cylindrique et à l'extérieur une géométrie différente de la géométrie cylindrique, en particulier une géométrie en forme de colonne polygonale.
